# EUROPEAN PATENT APPLICATION

(11) **EP 1 884 186 A1**
(43) Date of publication of application: **06.02.2008**
(21) Application number: 07014086.8
(22) Date of filing: 18.07.2007
(51) Int. Cl.: A61B 1/01

(54) **Endoscopic insertion aid**

(30) Priority: 01.08.2006 JP 2006210067
(71) Applicant: OLYMPUS MEDICAL SYSTEMS CORP., Shibuya-ku Tokyo 151-0072 (JP)
(72) Inventor: Matsui, Raifu, Hachioji-shi Tokyo 192-8512 (JP); Matsuura, Noboyuki, Hachioji-shi Tokyo 192-8512 (JP); Takase, Seisuke, Hachioji-shi Tokyo 192-8512 (JP); Kimura, Hidenobu, Hachioji-shi Tokyo 192-8512 (JP); Yoshida, Takatoshi, Hachioji-shi Tokyo 192-8512 (JP)
(74) Representative: von Hellfeld, Axel

(57) **Abstract**

An endoscopic insertion aid (14) includes a hollow member (62) having a communication path which provides communication between openings at the distal and proximal ends thereof. The hollow member includes a hollow member, an exposure portion and a guide portion. The hollow member insertion portion is inserted into a body cavity, having distal and proximal ends. The exposure portion is provided at the proximal end of the hollow member insertion portion and is exposed outside the body. The guide portion is provided in at least the exposure portion of the hollow member insertion portion and the exposure portion. The guide portion allows a distal end (32) of an insertion section (22) of an endoscope (12) to pass through the communication path laterally from the hollow member (62) and project from the opening at the distal end of the hollow member.

## Description

The present invention relates to an endoscopic insertion aid used to aid in the insertion of an insertion section of an endoscope into a body cavity.

An endoscopic insertion aid such as an overtube is disclosed in Jpn. Pat. Appln. KOKAI Publication No. 2002-301019.

The endoscopic insertion aid is used when an insertion section of an endoscope is inserted, for example, per anum into a desired region of the large intestine or when the insertion section of the endoscope inserted into the desired region is taken out of a body cavity and an insertion portion of a different endoscope is inserted into the desired region of the large intestine. In order to introduce and insert the different endoscope into a desired region within the body cavity through the overtube, the insertion section longer than the entire length of a hollow member of the overtube is inserted from an opening at the proximal end of the hollow member of the overtube to an opening at the distal end thereof.

However, the proximal side of the tube member of the overtube may project a long way from, for example, the anus depending on an observed region or a treatment target region. In such a case, the proximal end of the insertion section of the endoscope further extends to a hand side through the opening at the proximal end of the overtube. Further, when an insertion section of a different endoscope is inserted into, for example, the large intestine through the overtube, it is troublesome to insert it from the opening at the proximal end of the overtube to the opening at the distal end of the overtube. Moreover, when the endoscope adjusted to the length of the overtube is used, the length of the insertion section of the endoscope has to be increased, which increases the weight of the endoscope and decreases the response of a bending portion of the insertion section in, for example, bending operation, resulting in decreased operability of the endoscope in some cases. Still further, when the endoscope is used so that an endoscopic treatment tool is inserted through a treatment tool insertion channel of the endoscope, the length of the treatment tool has to be increased, which may also decrease the response of operation and decrease the operability of the treatment tool.

This invention has been made to solve such problems, and is directed to provide an endoscopic insertion aid which enables an endoscope optimum for an observed region ranging, for example, from the anus to be selectively used independently of the length of the endoscopic insertion aid such as an overtube and which can maintain the operability of the endoscope and an endoscopic treatment tool.

An endoscopic insertion aid according to this invention includes a hollow member. The hollow member having distal and proximal ends and a communication path which provides communication between openings at the distal and proximal ends thereof. The hollow member includes: a hollow member insertion portion, an exposure portion and a guide portion. The hollow member insertion portion inserted into a body cavity, having distal and proximal ends. The exposure portion is provided at the proximal end of the hollow member insertion portion and is exposed outside the body. The guide portion is provided in at least the exposure portion of the hollow member insertion portion and the exposure portion. And the guide portion allows a distal end of an insertion section of an endoscope to pass through the communication path laterally from the hollow member and project from the opening at the distal end of the hollow member.

The invention can be more fully understood from the following detailed description when taken in conjunction with the accompanying drawings, in which:
FIG. 1 is a schematic diagram showing an endoscopic system according to a first embodiment of the present invention;
FIG. 2A is a schematic diagram showing an overtube in the endoscopic system according to the first embodiment;
FIG. 2B is a schematic sectional view along the 2B-2B line of the overtube shown in FIG. 2A in the endoscopic system according to the first embodiment;
FIG. 3 is a schematic diagram showing how an insertion section of an endoscope is inserted from a slit of a hollow member of the overtube in the endoscopic system according to the first embodiment to the distal end of the hollow member;
FIG. 4 is a schematic diagram showing a modification (without a balloon) of the overtube in the endoscopic system according to the first embodiment;
FIG. 5 is a schematic diagram showing how the insertion section of the endoscope and the overtube are per anum inserted into the large intestine by use of the endoscopic system according to the first embodiment;
FIG. 6A is a schematic diagram showing how the distal end of the insertion section of the endoscope is per anum inserted from the rectum β to the vicinity of the sigmoid colon Υ;
FIG. 6B is a schematic diagram showing how the overtube is moved along the insertion section of the endoscope toward its distal side when the insertion of the insertion portion into the intestinal tract α has become difficult;
FIG. 6C is a schematic diagram showing how the balloon is slowly inflated so that the balloon is held by the inner surface of the intestinal wall when the balloon is positioned in the vicinity of the distal end of the insertion portion;
FIG. 7A is a schematic diagram showing how the overtube and the insertion portion are drawn together to a hand side to shorten the bent intestinal tract α while the balloon is being held by the inner surface of the intestinal wall;
FIG. 7B is a schematic diagram showing how the insertion section of the endoscope is advanced, with respect to the overtube, to a place where it can be inserted, for example, to the vicinity of the descending colon δ so that the position of the overtube is fixed by the balloon to insert the insertion section of the endoscope into the descending colon δ;
FIG. 7C is a schematic diagram showing how the overtube is moved along the insertion section of the endoscope toward its distal side;
FIG. 8A is a schematic diagram showing how the balloon is again inflated so that the balloon is held by the intestinal wall;
FIG. 8B is a schematic diagram showing how the overtube and the insertion portion are drawn together to the hand side to bring the intestinal tract α into a nearly linear shape while shortening the bent place of the intestinal tract α;
FIG. 8C is a schematic diagram showing how to bring the intestinal tract α into a nearly linear shape while shortening the bent place of the intestinal tract α so that the insertion section of the endoscope is moved to a far side;
FIG. 9 is a schematic diagram showing how the insertion section of the endoscope and the overtube are orally inserted into the small intestine by use of the endoscopic system according to the first embodiment;
FIG. 10A is a schematic diagram showing an overtube in an endoscopic system according to a second embodiment of the present invention;
FIG. 10B is a schematic sectional view along the 10B-10B line of the overtube shown in FIG. 10A in the endoscopic system according to the second embodiment;
FIG. 10C is a schematic sectional view showing a modification of the schematic sectional view along the 10B-10B line of the overtube shown in FIG. 10A in the endoscopic system according to the second embodiment;
FIG. 11 is a schematic diagram showing an overtube in an endoscopic system according to a third embodiment of the present invention;
FIG. 12A is a schematic diagram showing a cross section of an overtube in an endoscopic system according to a fourth embodiment of the present invention;
FIG. 12B is a schematic diagram showing a cross section of the overtube in the endoscopic system according to the fourth embodiment;
FIG. 13A is a schematic diagram showing an overtube in an endoscopic system according to a fifth embodiment;
FIG. 13B is a schematic longitudinal sectional view showing how an insertion section of an endoscope is inserted from a through-hole laterally provided in a hollow member of the overtube shown in FIG. 13A into the hollow member;
FIG. 14 is a schematic diagram showing an overtube in an endoscopic system according to a sixth embodiment of the present invention;
FIG. 15A is a schematic diagram showing an overtube in an endoscopic system according to a seventh embodiment of the present invention;
FIG. 15B is a schematic sectional view along the 15B-15B line in FIG. 15A; and
FIG. 16 is a schematic diagram showing how an insertion section of an endoscope is inserted from a slot in a hollow member of an overtube in the endoscopic system according to the seventh embodiment to the distal end of the hollow member.

A best mode of carrying out this invention will hereinafter be described with reference to the drawings.

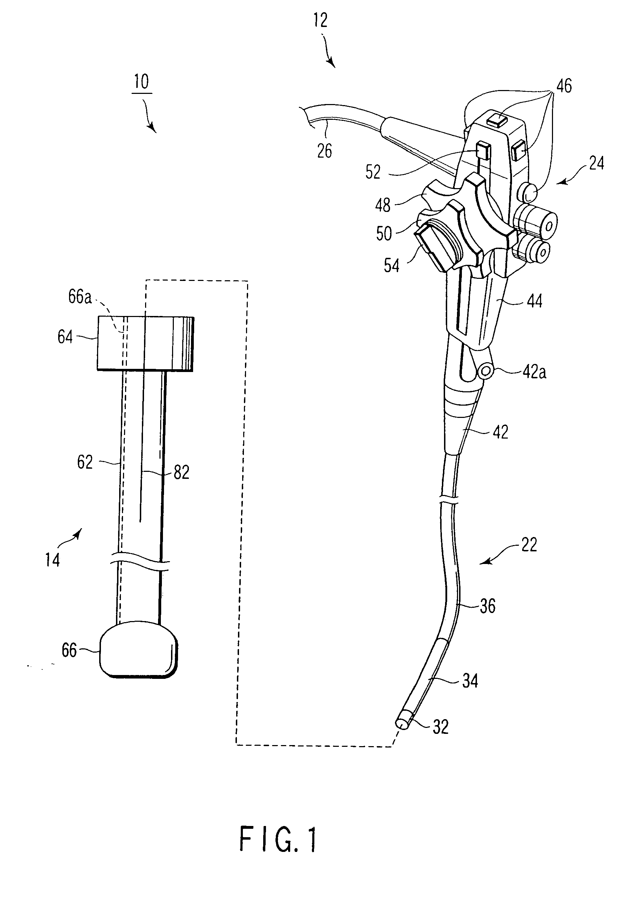

A first embodiment will be described using FIGS. 1 to 9.

As shown in FIG. 1, an endoscopic system 10 according to this embodiment includes an endoscope 12 and an overtube (endoscopic insertion aid) 14.

The endoscope 12 includes an elongated insertion section 22, and an operation portion 24 connected to a proximal end of the insertion section 22. One end of a universal cable 26 capable of transmitting illumination light from an unshown light source unit and various signals extends at the proximal end of the operation portion 24.

The insertion section 22 includes a rigid distal portion 32, a bending portion 34 capable of vertically and horizontally bending, and a long and flexible tube portion 36.

The rigid distal portion 32 is disposed at a most distal position of the insertion section 22. This rigid distal portion 32 is provided with a forceps opening communicating with an illumination optical system, an observation optical system such as a solid-state image sensing device, and a treatment tool insertion channel, and also provided with a nozzle for supplying air into the body cavity and water to an observation lens (both the forceps opening and the nozzle are not shown). The treatment tool insertion channel communicates with a treatment tool insertion hole (not shown) of the operation portion 24.

The distal end of the bending portion 34 is coupled to the proximal end of the rigid distal portion 32. The distal end of the flexible tube portion 36 is coupled to the proximal end of the bending portion 34. The distal end of the operation portion 24 is coupled to the proximal end of the flexible tube portion 36. That is, the distal end of the operation portion 24 is coupled to the proximal end of the insertion section 22.

A support portion 42 supporting the proximal end of the flexible tube portion 36 is provided at the distal end of the operation portion 24. The distal end of the support portion 42 is formed to taper toward the proximal end of the flexible tube portion 36 of the insertion section 22. A grip 44 for an operator is provided at the proximal end of the support portion 42. This grip 44 is provided with a remote switch 46 for the remote control of an unshown image recorder such as a VTR, an unshown camera control unit, etc.

Bending operation knobs 48 and 50 which are rotated by the operator are provided at the proximal end of the grip 44. When these bending operation knobs 48 and 50 are operated, the above-mentioned bending portion 34 bends in directions to deviate from a direction along the longitudinal axis of the flexible tube portion 36, for example, in vertical and horizontal directions. In addition, the operation knob indicated by 48 is designed for the vertical direction, and the operation knob indicated by 50 is designed for the horizontal direction.

At a position adjacent to the one bending operation knob 48, there is provided a bend fixing lever 52 for fixing the bending operation knob 48 at a desired position and fixing the bending portion 34 in a desired bending amount. This lever 52 is also operated to cancel the fixing of the bending portion 34. That is, this lever 52 is operated to fix the bending portion 34 in a desired state and to cancel the fixing to bring the bending operation knob 48 into a movable state.

The other bending operation knob 50 is also provided with a bend fixing lever 54 as is the bending operation knob 48. This lever 54 is also operated to cancel the fixing of the bending portion 34. That is, this lever 54 is operated to fix the bending portion 34 in a desired state and to cancel the fixing to bring the bending operation knob 50 into a movable state.

In order to facilitate the insertion of the insertion section 22 of the endoscope 12 having such a configuration, the endoscopic overtube 14 shown in FIG. 1 is used so that it is attached to a part of the insertion section 22.

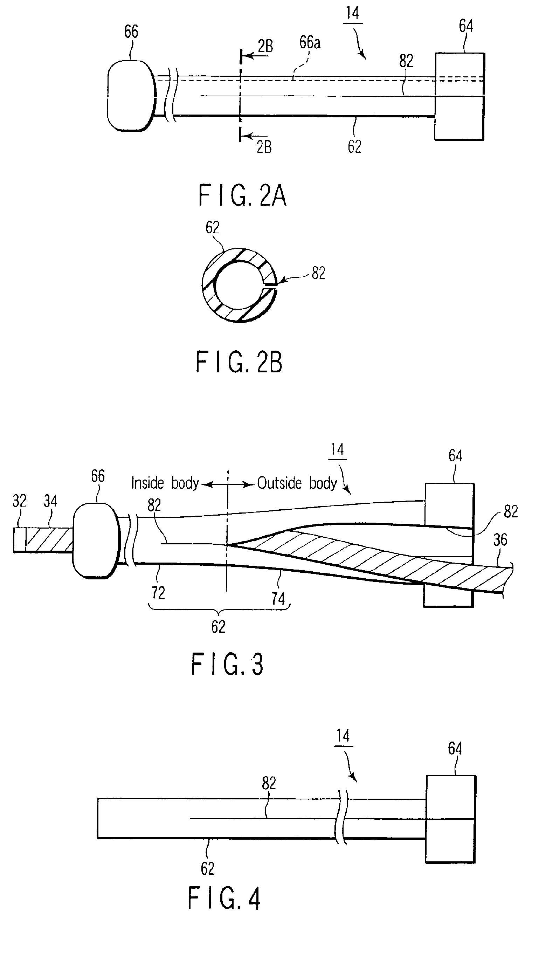

As shown in FIGS. 2A and 2B, in the overtube 14, there are integrally formed a hollow member 62 formed of, for example, silicone to have a cylindrical shape, and a grip portion 64 provided at the proximal end of the hollow member 62. The distal end and the proximal end (grip portion 64) of the hollow member 62 are open, and a communication path (insertion path) is formed therebetween.

Furthermore, an inflatable/deflatable balloon 66 is disposed on the outer peripheral surface at the distal end of the hollow member 62. This balloon 66 is connected to a device (not shown) for supplying/discharging a gas (fluid) through a pipeline 66a provided in the hollow member 62 and the grip portion 64.

The hollow member 62 is inserted into the body cavity and therefore has the flexibility to bend in accordance with the bending of the bending portion 34 of the insertion section 22 of the endoscope 12. Moreover, when the flexible tube portion 36 is bent, the hollow member 62 is bent accordingly. The overtube 14 has a length of several meters in the hollow member 62 when it is designed for, for example, the large intestine.

As shown in FIG. 3, this hollow member 62 includes a hollow member insertion portion 72 which is a part inserted into the body cavity (in the body), and an exposure portion 74 exposed outside the body cavity (outside the body). The exposure portion 74 is provided at the proximal end of the hollow member insertion portion 72. The exposure portion 74 is a part located outside the body when the hollow member insertion portion 72 is inserted into the body cavity. That is, if the entire length of the hollow member 62 is inserted into the body cavity, the entire hollow member 62 serves as the hollow member insertion portion 72. In this case, no exposure portion 74 exists.

The grip portion 64 is formed to diametrically outwardly project. This grip portion 64 serves as a stopper for preventing extra insertion when the exposure portion 74 of the hollow member 62 is inserted into the body cavity.

Furthermore, a slit (guide portion) 82 is formed in the hollow member 62 along its longitudinal direction. This slit 82 is formed to extend from a position separate from the distal end of the hollow member 62 at a proper distance to the grip portion 64 through the proximal end of the hollow member 62. Thus, an opening (guide portion) is formed in the overtube 14 from a proper part in the side surface of the hollow member 62 to the proximal side thereof.

Next, the function of the endoscopic system 10 according to this embodiment will be described.

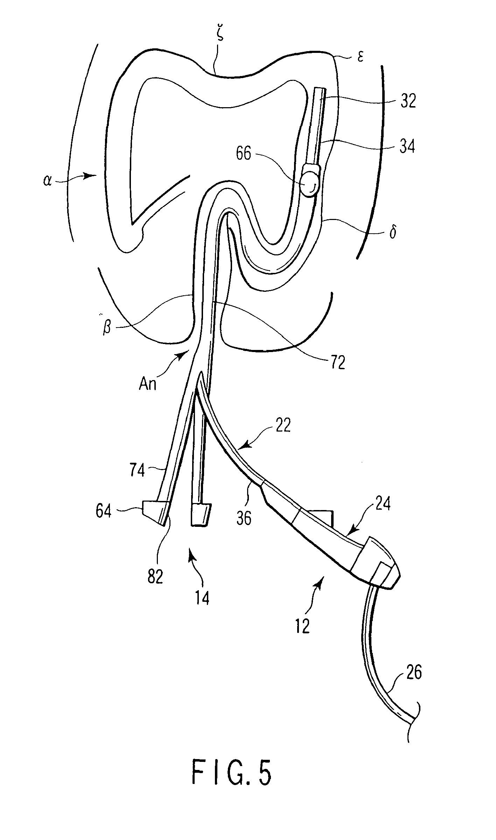

First, the overtube 14 is externally inserted into the insertion section 22 of the endoscope 12. That is, the insertion section 22 of the endoscope 12 is inserted through the communication path of the overtube 14. Then, the distal end of the insertion section 22 of the endoscope 12 is made to project out of the distal end of the hollow member 62 of the overtube 14. The distal end of the insertion section 22 of the endoscope 12 in this state is inserted from the side of the anus An into the intestinal tract α shown in FIG. 5. At this point, the flexible tube portion 36 of the insertion section 22 of the endoscope 12 extends from the slit 82 of the hollow member 62 of the overtube 14. Thus, the operation portion 24 can be easily gripped at a desired position, and desired operation performance is maintained.

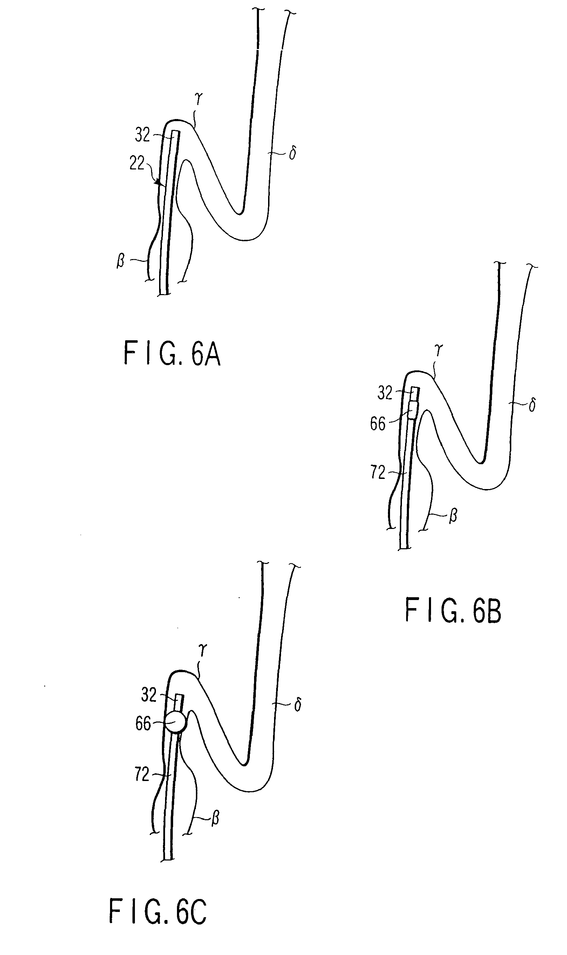

As shown in FIG. 6A, the distal end of the insertion section 22 can generally be inserted from the rectum β to the vicinity of the sigmoid colon Υ. When the insertion of the insertion section 22 into the intestinal tract α becomes difficult, the overtube 14 is moved along the insertion section 22 of the endoscope 12 toward its distal side (see FIG. 6B). As shown in FIG. 6C, when the balloon 66 is positioned in the vicinity of the distal end of the insertion section 22, the balloon 66 is slowly inflated so that the balloon 66 is held by the inner surface of the intestinal wall.

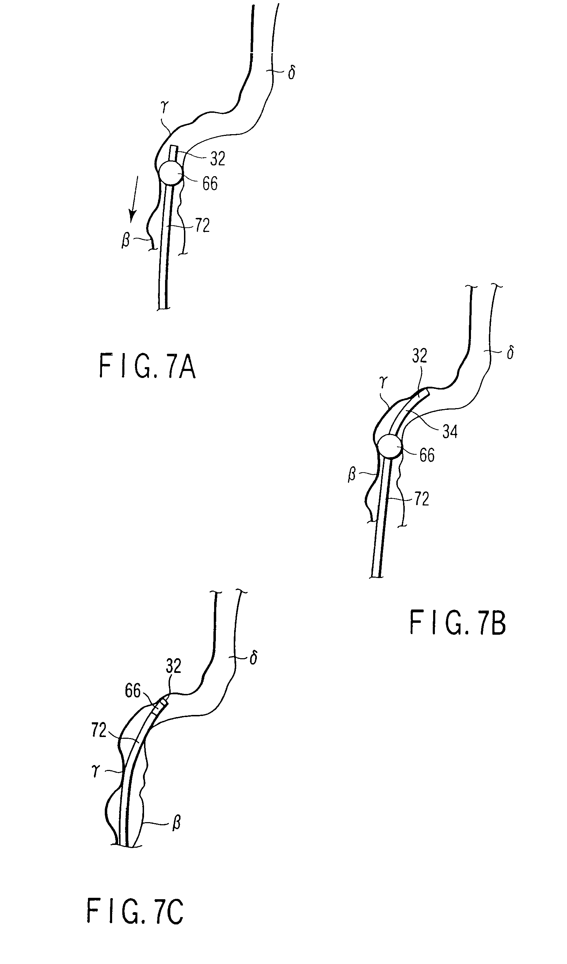

The overtube 14 and the insertion section 22 are drawn together to the hand side while the balloon 66 is being held by the inner surface of the intestinal wall. When the overtube 14 and the insertion section 22 are drawn, the bent intestinal tract α shrinks as shown in FIG. 7A. While this state is maintained, the insertion section 22 of the endoscope 12 is advanced, with respect to the overtube 14, to a place where it can be inserted, for example, to the vicinity of the descending colon δ, as shown in FIG. 7B. That is, the position of the overtube 14 is fixed by the balloon 66 to insert the insertion section 22 of the endoscope 12 into the descending colon δ. Then, the balloon 66 is deflated.

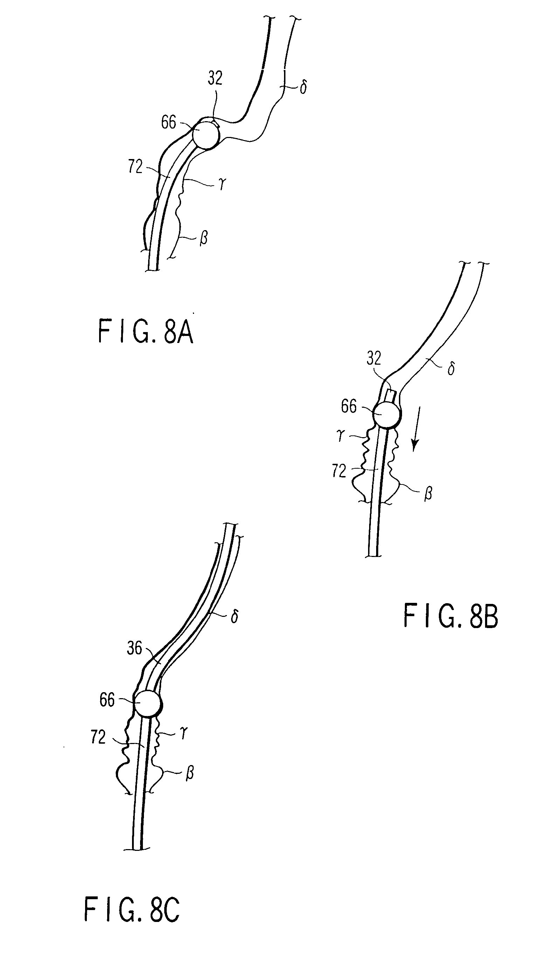

While the balloon 66 is deflated, the overtube 14 is moved along the insertion section 22 of the endoscope 12 toward its distal side as shown in FIG. 7C. Then, the balloon 66 is again inflated so that the balloon 66 is held by the intestinal wall, as shown in FIG. 8A. Then, the overtube 14 and the insertion section 22 are drawn together to the hand side, as shown in FIG. 8B. Such operation is repeated to bring the bent place of the intestinal tract α into a nearly linear shape while shortening this place, and then the insertion section 22 of the endoscope 12 is further advanced (see FIG. 8C). The overtube 14 and the insertion section 22 of the endoscope 12 are repeatedly operated in this manner, such that the insertion section 22 of the endoscope 12 is inserted from the left colic flexure ε even further than the transverse colon ξ.

Then, an endoscopic treatment tool is inserted from a forceps plug 42a of the endoscope 12 into an unshown treatment tool insertion channel to conduct a desired treatment.

For example, when an endoscope having a different function (e.g., an ultrasonic endoscope) is used (when the endoscope 12 is replaced), the insertion section 22 of the endoscope 12 is slowly pulled out, for example, while the balloon 66 is inflated. Thus, the distal end of the hollow member 62 of the overtube 14 holds its position with respect to the intestinal tract α. Then, the insertion section 22 of the different endoscope 12 is inserted into the hollow member insertion portion 72 from the slit 82 provided in the exposure portion 74 of the hollow member 62 of the overtube 14 to make the distal end of the insertion section 22 project. That is, the insertion section 22 of the endoscope 12 is advanced along the overtube 14 and inserted into the position where the distal end of the insertion section 22 of the former endoscope 12 has been located. Thus, the slit 82 serves as a guide portion for guiding the insertion of the insertion section 22 of the endoscope 12 into the communication path of the hollow member 62.

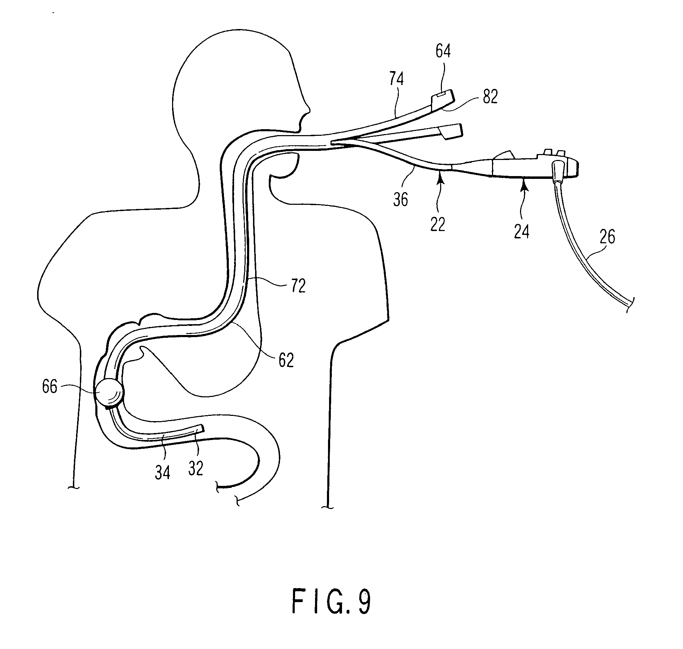

In addition, the endoscopic system 10 according to this embodiment is not limited to the use in the observation, etc. of the large intestine and the small intestine by per anum inserting the insertion section 22 of the endoscope 12, but the endoscopic system 10 according to this embodiment can also be suitably used when the endoscope 12 is orally introduced into the body of a patient, for example, as shown in FIG. 9. In this case, the overtube 14 can protect the inner wall of the body cavity against friction caused by the movement (back-and-forth operation) of the insertion section 22 of the endoscope 12 with respect to the inner wall of the body cavity.

As described above, the following effects can be obtained according to this embodiment.

The insertion section 22 of the endoscope 12 can be introduced into the hollow member 62 from the slit 82 when the insertion section 22 of the endoscope 12 is inserted into a desired position by use of the overtube 14. Therefore, the position for inserting the insertion section 22 of the endoscope 12 can be located closer to the desired position as compared with the case where the insertion section 22 is passed through the entire length of the overtube 14. That is, the length of the insertion of the insertion section 22 of the endoscope 12 into the hollow member 62 of the overtube 14 can be minimized. Thus, the operation portion 24 can be disposed closer to the desired position. Consequently, satisfactory operability of the endoscope 12 can be maintained. Moreover, it is possible to prevent the influence of the gravity and reaction force attributed to parts (e.g., the proximal end of the hollow member 62 and the grip portion 64) located closer to the proximal side than the position where the insertion section 22 of the endoscope 12 is inserted into the hollow member 62.

Furthermore, when the endoscope 12 is reinserted or when the different endoscope 12 is used, the insertion section 22 of the endoscope 12 has only to be led to the distal end of the hollow member insertion portion 72 from the slit 82 provided in the exposure portion 74, so that the insertion length of the insertion section 22 of the endoscope 12 can be reduced.

Moreover, it is also possible to use the overtube 14 whose entire length is substantially equal to or longer than the length of the insertion section 22 of the endoscope 12. For example, it is possible to easily use even the endoscope 12 whose insertion section 22 is shorter than the entire length of the overtube 14 when the distance from the anus Aₙ to an observation position or treatment position is short. That is, the distance between the operation portion 24 and the bending portion 34 of the insertion section 22 can be reduced, so that the response and operability in the bending operation can be improved, and the lighter endoscope 12 can be used. Therefore, the operability of the endoscope 12 can be improved. It is thus possible to use the common overtube 14 even for the endoscope 12 of a different kind as long as the inside diameter of the overtube 14 is greater than the outside diameter of the insertion section 22 of the endoscope 12. Therefore, the kinds of overtube 14 can be reduced.

In addition, while the example of the overtube 14 provided with the balloon 66 at the distal end of the hollow member 62 has been described in this embodiment, the overtube 14 which is not provided with the balloon 66 on the outer peripheral surface at the distal end of the hollow member 62 can also contribute to the improvement of the operability of the endoscope 12.

Furthermore, this embodiment has been described on the assumption that the slit 82 is provided in advance in at least part of the hollow member 62 and the grip portion 64, but it is also possible to provide a cut which does not communicate the inside of the hollow member 62 to the outside thereof (e.g., perforations provided at the same position as the slit 82, or a thin portion separably and linearly formed at the same position as the slit 82). The cut in this case can be easily made only in the part where the insertion section 22 of the endoscope 12 is inserted into the communication path of the hollow member 62 or can be made over the entire hollow member 62. That is, the cut can be used so that part of the cut portion is removed as necessary in use, or the entire cut such as the slit 82 can be used.

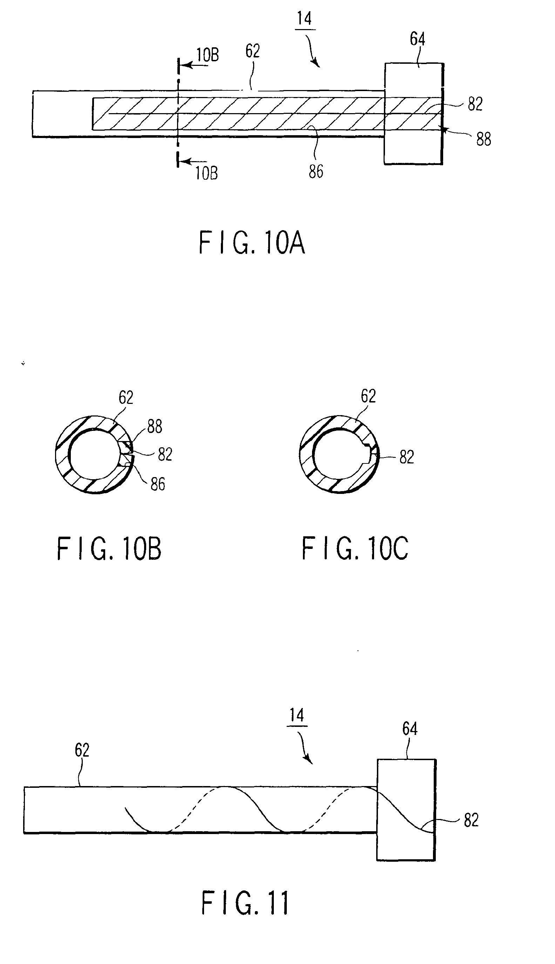

Next, a second embodiment will be described using FIGS. 10A to 10C. This embodiment is a modification of the first embodiment, so that the same signs are assigned to the same members as those described in the first embodiment, and these members are not described in detail.

As shown in FIGS. 10A and 10B, a slot 86 is formed in a hollow member 62. A flexible portion 88 is formed in the slot 86 using a member more flexible than the member forming the hollow member 62. A slit 82 is formed in the flexible portion 88 axially along the hollow member 62. The formation of the slit 82 makes it possible to obtain functions and effects similar to those in the first embodiment.

Furthermore, since the slit 82 is formed in the flexible portion 88, the insertion section 22 of the endoscope 12 can be easily inserted into the inside (communication path) of the hollow member 62 through the slit 82. That is, the slit 82 can be easily opened/closed when the insertion section 22 of the endoscope 12 is inserted/removed or when the insertion section 22 of the endoscope 12 is moved axially along the hollow member 62. Moreover, the part of the slit 82 can prevent great force from being applied to the insertion section 22 of the endoscope 12. Even when force is applied to the distal end of the slit 82, it is possible to prevent the slit 82 from being unintentionally formed on the distal side of the hollow member 62 further than the distal side of the slot 86.

In addition, while the provision of the flexible portion 88 having the slit 82 in the slot 86 has been described in this embodiment, it is also possible to provide a thinner part having the slit 82 than other parts instead of the flexible portion 88 to bring the same functions, as shown in FIG. 10C.

In this case, the slit 82 can be easily opened/closed when the insertion section 22 of the endoscope 12 is inserted/removed or when the insertion section 22 of the endoscope 12 is moved axially along the hollow member 62. Moreover, since the slit 82 is flexibly formed, it is possible to prevent great force from being applied to the insertion section 22 of the endoscope 12 in the part of the slit 82.

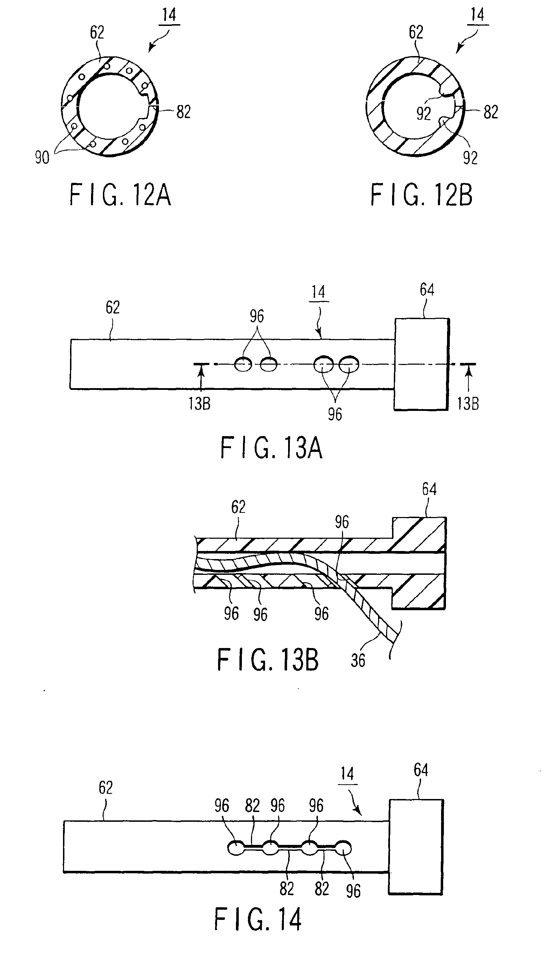

Next, a third embodiment will be described using FIG. 11. This embodiment is a modification of the first embodiment, so that the same signs are assigned to the same members as those described in the first embodiment, and these members are not described in detail.

As shown in FIG. 11, a slit 82 is formed in a hollow member 62 and a grip portion 64. This slit 82 is spirally formed to be directed toward the hollow member 62 from the proximal end of the grip portion 64. The distal end of the slit 82 is located at a proper position between the distal end and the proximal end of the hollow member 62.

When the insertion section 22 of the endoscope 12 is rotated with respect to the overtube 14, the insertion section 22 of the endoscope 12 can be moved along the slit 82. Therefore, this modification is suitably used when such operation is to be performed, and the operability of the endoscope 12 can be improved.

Next, a fourth embodiment will be described using FIGS. 12A and 12B. This embodiment is a modification of the first embodiment, so that the same signs are assigned to the same members as those described in the first embodiment, and these members are not described in detail.

As shown in FIG. 12A, a plurality of wires (reinforcing members) 90 are circumferentially arranged side by side at predetermined intervals in a hollow member 62. That is, the plurality of wires 90 are disposed axially along the hollow member 62. The wires 90 are formed of, for example, plastic or steel thin lines, and are buried axially along the hollow member 62. Thus, it is possible to prevent the buckling of the overtube 14.

As shown in FIG. 3, at least part (the distal end) of a slit 82 formed in the hollow member 62 may be placed in the body cavity. That is, the slit 82 may be provided in the hollow member insertion portion 72. In such a case, the overtube 14 may be twisted, or the overtube 14 may rotate in accordance with the rotation of the insertion section 22 of the endoscope 12. When the overtube 14 (the hollow member 62) is thus twisted, it is possible to prevent the slit 82 from easily opening/closing in, for example, the body cavity in accordance with the twist. That is, it is possible to prevent the slit 82 from unintentionally opening. Therefore, the hollow member 62 can be reinforced in a direction to close the slit 82, and the insertion section 22 of the endoscope 12 can be prevented from taking off in the hollow member insertion portion 72.

Furthermore, as shown in FIG. 12B, a thick portion (reinforcing member) 92 is provided around the slit 82. This thick portion 92 is formed for reinforcement to prevent the opening/closing of the slit 82. Thus, it is possible to prevent the slit 82 from unintentionally opening in, for example, the body cavity.

Next, a fifth embodiment will be described using FIGS. 13A and 13B. This embodiment is a modification of the first embodiment, so that the same signs are assigned to the same members as those described in the first embodiment, and these members are not described in detail.

As shown in FIG. 13A, a plurality of through-holes (guide portions) 96 are formed in a hollow member 62 axially along the hollow member 62. For example, a pair of through-holes 96 is formed to have the same diameter. Thus, the through-holes 96 at the distal end of the hollow member 62 are used or the through-holes 96 at the proximal end of the hollow member 62 are used depending on the position where the hollow member 62 is inserted (the relation between the hollow member insertion portion 72 and an exposure portion 74).

As shown in FIG. 13B, the through-holes 96 are formed at a slant with respect to the direction perpendicular to the axis of the hollow member 62. The part of the through-hole 96 on the outer peripheral side of the hollow member 62 is in proximity to the grip portion 64, while the part of the through-hole 96 on the inner peripheral side of the hollow member 62 is in proximity to the distal end of the hollow member 62. Thus, the insertability of the insertion section 22 of the endoscope 12 can be improved. That is, the distal end of the insertion section 22 of the endoscope 12 can be easily led to the distal side of the hollow member 62.

In addition, while FIGS. 13A and 13B show the through-holes 96 that are formed in line axially along the hollow member 62, it is also possible to additionally form through-holes (not shown) at the opposite positions. Further, it is also possible to spirally provide the through-holes 96 of the hollow member 62 as described in the third embodiment (see FIG. 11). Still further, the positions of the through-holes 96 of the same diameter do not have to be adjacent to each other as shown in FIG. 13B.

Next, a sixth embodiment will be described using FIG. 14. This embodiment is a modification of the first embodiment, so that the same signs are assigned to the same members as those described in the first embodiment, and these members are not described in detail.

As shown in FIG. 14, a plurality of through-holes 96 are linked by slit 82 in a hollow member 62. Thus, after the insertion section 22 of the endoscope 12 is moved to one of the through-holes 96, the hollow member 62 can be elastically deformed by use of the slit 82 to move the insertion section 22 of the endoscope 12 into the adjacent through-hole 96. Therefore, when the insertion section 22 of the endoscope 12 is inserted in the distal through-hole 96 provided in the exposure portion 74 of the hollow member 62 and this distal through-hole 96 is then inserted into the body cavity and changes into the hollow member insertion portion 72, the insertion section 22 of the endoscope 12 can be moved to the rear through-hole 96 while the insertion section 22 of the endoscope 12 is being inserted through the overtube 14. Therefore, it is always possible to select and use the optimum through-hole 96.

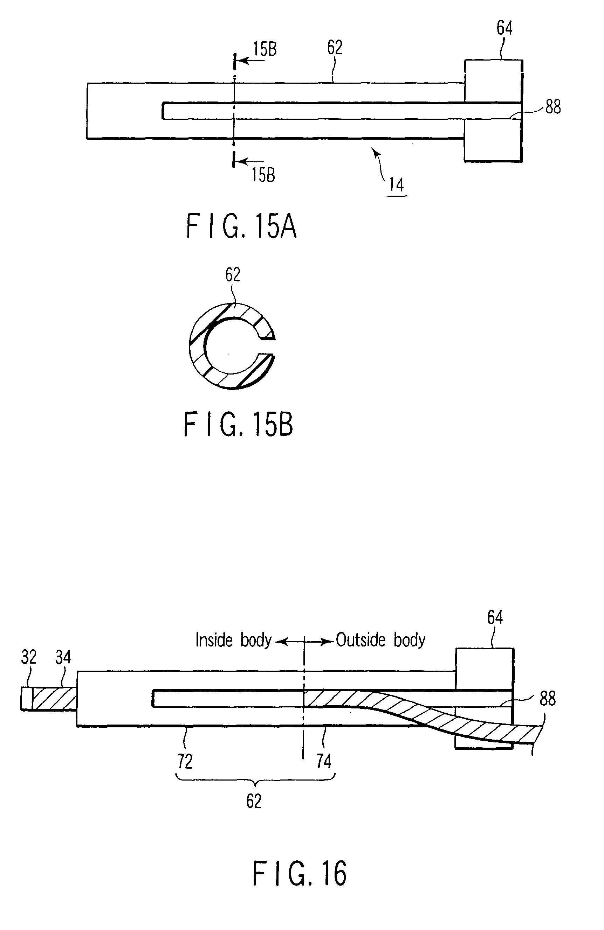

Next, a seventh embodiment will be described using FIGS. 15A to 16. This embodiment is a modification of the first embodiment, so that the same signs are assigned to the same members as those described in the first embodiment, and these members are not described in detail.

As shown in FIGS. 15A and 15B, a slot 86 is formed in a hollow member 62 instead of the slit 82 (see FIGS. 2A and 2B). This slot 86 is formed to have a width substantially equal to the outside diameter of an insertion section 22 of a proper endoscope 12.

In addition, the distal end of this slot 86 is rectangularly formed, but may also be semicircularly formed. In this case, it is possible to prevent the concentration of stress on the distal end of the slot 86.

Furthermore, the slot 86 does not have to be provided up to the proximal end (the grip portion 64) of the hollow member 62. That is, the slot 86 has only to be provided such that the insertion section 22 can be inserted from the slot 86 and moved over a proper distance.

The insertion section 22 of the endoscope 12 can be easily inserted into the inside of the hollow member 62 through the slot 86 of the hollow member 62. At this point, the insertion section 22 of the endoscope 12 shown in FIG. 16 can be easily moved along the slot 86 of the hollow member 62. Thus, the position for inserting the insertion section 22 of the endoscope 12 into the hollow member 62 can be easily changed along with the change from the exposure portion 74 to the hollow member insertion portion 72.

In addition, the slit 82 and the slot 86 are provided from the position properly away from the distal end of the hollow member 62 of the overtube 14 to the grip portion 64 in the first to seventh embodiments described above. However, the slit 82 and the slot 86 may also be formed over the entire length of the overtube 14 from the distal end of the hollow member 62 to the grip portion 64. That is, the cross section extending from the distal end of the hollow member 62 of the overtube 14 to the grip portion 64 may also be substantially C-shaped.

## Claims

1. An endoscopic insertion aid (14) **characterized by** comprising:
a hollow member (62) having distal and proximal ends and a communication path which provides communication between openings at the distal and proximal ends thereof,
the hollow member including:
a hollow member insertion portion (72) inserted into a body cavity, having distal and proximal ends;
an exposure portion (74) which is provided at the proximal end of the hollow member insertion portion and which is exposed outside the body; and
a guide portion (82; 86; 88; 96) which is provided in at least the exposure portion of the hollow member insertion portion and the exposure portion and which allows a distal end (32) of an insertion section (22) of an endoscope (12) to pass through the communication path laterally from the hollow member (62) and project from the opening at the distal end of the hollow member.

2. The endoscopic insertion aid (14) according to claim 1, **characterized in that**
the guide portion (82) includes a slit which is formed in at least part of the side surface of the hollow member (62) and which is formed to be directed toward the proximal side of the exposure portion (74).

3. The endoscopic insertion aid (14) according to claim 2, **characterized in that**
a part around the slit (82) is thinner than the exposure portion (74).

4. The endoscopic insertion aid (14) according to claim 2 or 3, **characterized in that**
a part around the slit (82) is more flexible than the exposure portion (74).

5. The endoscopic insertion aid (14) according to any one of claims 2 to 4, **characterized in that**
a reinforcing portion (92) which prevents the opening/closing of the slit is disposed around the slit (82).

6. The endoscopic insertion aid (14) according to claim 1, **characterized in that**
the guide portion (82; 88) includes a flexible portion (88) formed in at least part of the side surface of the hollow member (62), and
the flexible portion includes a slit (82) formed to be directed toward the proximal side of the exposure portion (74).

7. The endoscopic insertion aid (14) according to claim 6, **characterized in that**
the flexible portion (88) is thinner than the exposure portion (74).

8. The endoscopic insertion aid (14) according to claim 6 or 7, **characterized in that**
the flexible portion (88) is more flexible than the exposure portion (74).

9. The endoscopic insertion aid (14) according to claim 1, **characterized in that**
the guide portion (96) includes a plurality of through-holes in at least part of the side surface of the hollow member (62).

10. The endoscopic insertion aid (14) according to claim 9, **characterized in that**
the guide portion includes, across the through-holes (96), a slit which is formed in at least part of the side surface of the hollow member (62) and which is formed to be directed toward the proximal side of the exposure portion (74).

11. The endoscopic insertion aid (14) according to claim 9 or 10, **characterized in that**
a part around the through-holes (96) is thinner than the exposure portion (74).

12. The endoscopic insertion aid (14) according to any one of claims 8 to 10, **characterized in that**
a part around the through-holes (96) is more flexible than the exposure portion (74).

13. The endoscopic insertion aid (14) according to claim 1, **characterized in that**
the guide portion (86) includes a slot (86) which is formed in at least part of the side surface of the hollow member (62) and which is formed to be directed toward the proximal side of the exposure portion (74), and
the slot has a width substantially equal to the outside diameter of the insertion portion (22) of the endoscope (12).

14. The endoscopic insertion aid (14) according to claim 13, **characterized in that**
a flexible portion (88) is disposed in the slot (86), and
a slit (82) is disposed in the flexible portion axially along the slot.

15. The endoscopic insertion aid (14) according to any one of claims 1 to 14, **characterized in that**
the guide portion (82; 86; 88; 96) is formed along the longitudinal direction of the hollow member (62).

16. The endoscopic insertion aid (14) according to any one of claims 1 to 15, **characterized in that**
the guide portion (82; 86; 88; 96) is spirally formed in the hollow member (62).

17. The endoscopic insertion aid (14) according to any one of claims 1 to 16, **characterized in that**
a reinforcing member (90) which prevents the buckling of the hollow member (62) is buried in the hollow member.
